# FASCICULE DE BREVET EUROPEEN

(11) **EP 0 580 829 B1**
(45) Date de publication et mention de la délivrance du brevet: **26.11.1997**
(21) Numéro de dépôt: 93902023.6
(22) Date de dépôt: 03.02.1993
(51) Int. Cl.: C12P 21/00, A61K 35/74

(54) **Extrait à base de protéines bactériennes, procédé pour sa préparation et composition pharmaceutique le contenant**
Aus bakteriellen Proteinen bestehender Extrakt, Verfahren zur dessen Herstellung und diesen Extrakt enthaltende pharmazeutische Zusammensetzung
Bacterial protein extract, process for preparing the same and pharmaceutical composition containing said extract

(30) Priorité: 14.02.1992 CH 450/92
(43) Date de publication de la demande: 02.02.1994
(73) Titulaire: LABORATOIRES OM S.A., 1217 Meyrin 2 (CH)
(72) Inventeur: BAUER, Jacques, CH-1025 Saint-Sulpice (CH); HIRT, Pierre, CH-1028 Préverenges (CH); SCHULTHESS, Adrian, CH-1268 Begnins (CH)
(74) Mandataire: Micheli & Cie
(86) Numéro de dépôt international: CH9300029
(87) Numéro de publication internationale: WO9316190

(56) Documents cités:
- FR-A- 2 396 018
- FR-A- 2 405 298

## Description

La présente invention se rapporte à un extrait de macromolécules bactériennes, plus particulièrement à un extrait à base de protéines bactériennes modifiées, c'est-à-dire dont certains acides aminés sont racémisés et les résidus asparagine et glutamine sont partiellement désaminés à un procédé pour la préparation de celui-ci et à une composition pharmaceutique contenant à titre d'ingrédient actif cet extrait.

On connaît déjà des produits bactériens ayant une activité thérapeutique qui sont obtenus par hydrolyse alcaline. Le brevet CH 633.188 de la même titulaire divulgue par exemple un concentré de lysats bactériens ayant des propriétés anti-infectieuses. Les lysats de chaque souche bactérienne sont obtenus par une hydrolyse alcaline progressive (pH 9-10) qui conduit à la destruction de la structure apparente des bactéries.

Or, les présents inventeurs ont constaté que l'on pouvait obtenir des extraits bactériens ayant divers effets immunopharmacologiques, par exemple une activité immunomodulatrice importante, grâce à un traitement alcalin différent qui permet de maintenir intacte la structure apparente des bactéries traitées. Ce traitement consiste plus particulièrement en une extraction alcaline qui met en oeuvre des conditions de pH élévé et surtout stable.

Ainsi, la présente invention a pour objet d'une part un extrait à base de protéines bactériennes modifiées et d'autre part un procédé pour la préparation de cet extrait protéinique, qui sont définis dans les revendications 1 et 4 respectivement.

L'invention a également pour objet une composition pharmaceutique contenant, à titre d'ingrédient actif, l'extrait à base de protéines bactériennes modifiées selon l'invention (ci-après dénommé: extrait protéinique).

Le traitement chimique de bactéries, par exemple au moyen de NaOH dilué, entraîne de profondes modifications des structures primaires, secondaires et tertiaires des protéines bactériennes. On peut ainsi observer la désamidation partielle de l'asparagine et de la glutamine, qui conduit respectivement à la formation de l'acide aspartique et de l'acide glutamique. Des racémisations partielles de plusieurs acides aminés, principalement de l'acide aspartique, de la sérine et de l'arginine, ont également lieu au cours de ce processus. L'ensemble de ces modifications physico-chimiques de la structure des protéines génère des polypeptides dont les propriétés amphioniques sont acides. Ces polypeptides possèdent tous de bas points isoélectriques compris entre 2,5 et 5,5, avec une concentration plus importante aux environs de 4,5. Ces changements structurels produisent ainsi des extraits protéiniques qui montrent des activités immunomodulatrices in vitro et in vivo.

Dans l'extrait protéinique selon l'invention, la somme pondérale des acides aminés constitutifs représente au moins 50 % (% poids lyophilisat), de préférence de 55 à 85 % dudit extrait protéinique, et la teneur en lipopolysaccharides (LPS) est de préférence inférieure à environ 2 x 10⁻³ % Le poids moléculaire des éléments constitutifs de l'extrait protéinique selon l'invention est compris entre 10.000 et 1.000.000. En outre, cet extrait protéinique comporte parmi les acides aminés protéiniques, certains de configuration D et L, une proportion prépondérante de résidus acides tels que l'acide aspartique et l'acide glutamique. Les principaux acides aminés racémisés sont la sérine entre 25 et 45 % de configuration D environ, l'acide aspartique entre 10 et 30 % environ et l'arginine entre 3 et 20 % environ. L'emploi de la terminologie "protéines modifiées" est lié, en autre, à la présence d'acides aminés de configuration D dans l'extrait protéinique, la configuration L étant présente dans les protéines natives.

D'autre part l'extrait protéinique selon l'invention peut comporter au plus environ 10⁻³ % de lipopolysaccharides, au plus environ 2 % d'acides aminés libres, au plus environ 8 % de glucides, au plus environ 4 % de sucres aminés et au plus environ 15 % d'acides désoxyribonucléiques.

En principe, n'importe quelle(s) souche(s) de bactérie(s) gram (+) ou gram(-) peut (peuvent) être utilisée(s) comme produit(s) de départ du procédé selon l'invention, comme des Escherichia coli ou l'une ou plusieurs des souches bactériennes décrites dans le brevet précité CH 633.188. A titre d'exemple, le procédé qui est décrit ci-après utilise une souche Escherichia coli, plus particulièrement la souche I-1147, déposée conformément au traité de Budapest le 3 octobre 1991 auprès de la Collection Nationale de Cultures de Microorganismes, Institut Pasteur, 25 rue du Dr. Roux, 75724 PARIS CEDEX 15 - FRANCE.

### Exemple.

### A. Etapes préliminaires.

Un inoculum primaire est préparé à partir de bactéries congelées qui sont revivifiées par une croissance alternée en milieu liquide, par exemple "Tryptic Soya Broth", et sur un milieu solide. Le volume de culture de l'inoculum est augmenté progressivement jusqu'à 1 L, dans un milieu de culture liquide usuel, et incubé à 37 °C avec agitation.

L'inoculum secondaire ainsi préparé est introduit dans un fermenteur de 20 L contenant 5 L d'un milieu nutritif usuel approprié. Le pH est ajusté et maintenu à 7,0, par exemple avec de l'ammoniaque 5 %, et la température réglée à 37 °C. La croissance bactérienne est effectuée sous aération (air) et agitation, avec un contrôle des conditions pour obtenir une pO₂ supérieure à 90 % de saturation. Un suivi de la densité optique à 700 nm est effectué. En fin de culture, les bactéries sont inactivées à la chaleur, par autoclavage (30 minutes à 120 °C) ou par flash-pasteurisation (90 secondes à 105 °C), et un prélèvement est effectué pour vérifier l'absence de germes revivifiables.

La suspension bactérienne est concentrée et les résidus de milieu de culture non consommés sont éliminés par centrifugation, ou de préférence par ultrafiltration tangentielle, par exemple avec un système d'ultrafiltration sur cartouches de polysulfone du type de celui de la société Filtron. Cette ultrafiltration est effectuée en deux étapes, la première consistant en une concentration de 5 à 10 fois (membranes de 30 kD à 0,3 µm), la seconde en un lavage par diafiltration avec du soluté physiologique (3 à 15 volumes de lavage).

Le résidu sec de la suspension bactérienne est déterminé, ce qui permet d'ajuster la biomasse de la préparation pour la soumettre ensuite à l'extraction alcaline. La suspension finale des bactéries est diluée dans du soluté physiologique et le poids sec final des bactéries est ajusté entre 2 et 20 g/L.

### B. Extraction alcaline.

Pour l'extraction alcaline proprement de, la concentration en NaOH de la suspension bactérienne est ajustée entre 0,01 et 1 %, ici à 0,1 %. L'extraction est effectuée entre 30 et 45 °C sous agitation, ici à environ 37 °C. Des prélèvements sont réalisés et un suivi analytique au cours du temps est effectué. Ce suivi comporte la mesure du pH, le dosage des protéines et des lipopolysaccharides. Les bactéries utilisées pour obtenir les lots I et II ont été concentrées et lavées (étape A) sur cartouche 30 kD, alors que celles pour le lot III l'ont été sur cartouche 1000 kD. Dans les présents exemples, la durée du processus d'extraction alcaline a été d'un peu moins de deux jours. Les résultats, concernant trois lots (I, II et III) obtenus à partir de la souche Escherichia coli précitée, sont mentionnés dans le Tableau 1.

**Tableau 1:**

| Suivi de l'extraction alcaline. | | | | | |
|---|---|---|---|---|---|
| Lots | Poids Sec (g/L) | Durée | pH | Protéines* (mg/L) | LPS** (mg/L) |
| I | 7,4 | 0 minute | 7,0 | 220 | >100 |
| | | 5 minutes | 12,3 | 910 | 100 |
| | | 22 heures | 12,3 | 1530 | 8,0 |
| | | 46 heures | 12,3 | 1850 | 1,0 |
| II | 7,4 | 0 minute | 7,0 | 250 | >100 |
| | | 5 minutes | 12,4 | 870 | 100 |
| | | 22 heures | 12,4 | 1580 | 10 |
| | | 46 heures | 12,3 | 1990 | 3,3 |
| III | 7,0 | 0 minute | 7,0 | 60 | >100 |
| | | 5 minutes | 12,4 | 710 | 100 |
| | | 22 heures | 12,4 | 1260 | 10 |
| | | 46 heures | 12,4 | 1640 | 1,0 |

| | | | | | |
|---|---|---|---|---|---|
| * Dosage des protéines selon la méthode Bradford Référence: Albumine Sérique de Boeuf (BSA) | | | | | |
| ** Dosage des LPS selon la méthode LAL Référence: LPS de Escherichia coli 0111:B4 | | | | | |

Ce suivi permet en outre à l'homme du métier de déterminer la durée appropriée du processus d'extraction alcaline en fonction du but recherché et en tenant compte du type de bactéries utilisées ainsi que des conditions de pH et de température. Cette durée est généralement comprise entre quelques heures et une semaine environ.

En ce qui concerne le pH, rappelons qu'une des caractéristiques du procédé selon l'invention consiste en la stabilité de celui-ci, qui se maintient entre 11 et 13, par exemple entre 12,0 et 12,5 avec une baisse au cours du processus d'extraction d'au plus 0,4. Dans le présent exemple, le pH était de 12,3 à 12,4 avec une variation maximale de 0,1 au cours de l'extraction.

Un avantage supplémentaire important du procédé selon l'invention est mis en évidence par les résultats figurant dans le Tableau 1. En effet, on constate que la teneur en lipopolysaccharides dans l'extrait protéinique obtenu est notablement réduite.

A la fin de l'extraction, et avant la purification de l'extrait protéinique, un contrôle microscopique des bactéries après coloration de gram est effectué, afin de s'assurer que les structures apparentes des bactéries sont intactes.

### C. Purification de l'extrait protéinique.

L'extrait protéinique, obtenu par le processus décrit sous (B), est fractionné par ultrafiltration; plus particulièrement, il est concentré 5 à 10 fois par ultrafiltration (1000 kD), et le principe actif est extrait par diafiltration avec de l'eau, par exemple. L'extrait protéinique est finalement concentré par ultrafiltration (10 kD).

Afin d'éliminer la majeure partie des endotoxines, plus particulièrement les LPS, l'extrait protéinique est soumis à une procédure de transfert de phase avec un détergent non-ionique. A titre d'exemple, cette procédure peut être effectuée en présence de "Triton® X-114" à 7 %, en chauffant à environ 60 °C sous agitation pendant environ 20 minutes. Après séparation des phases, la phase inférieure (Triton® + LPS) est éliminée. La phase aqueuse supérieure contenant l'extrait protéinique est récoltée et le pH est ajusté à 7.

Cette phase aqueuse est soumise à une chromatographie échangeuse d'anions pour réduire encore la teneur en LPS et éliminer le détergent non-ionique. On peut utiliser pour cette chromatographie un gel DEAE-Sepharose® par exemple. Plus particulièrement, la phase aqueuse contenant l'extrait protéinique est d'abord adsorbée sur l'échangeur d'anions, puis les molécules non retenues sont éliminées et un lavage à bas pH élimine les impuretés. Après équilibrage du gel à pH neutre, l'extrait protéinique est élué par augmentation de la force ionique, puis l'éluat est concentré par ultrafiltration et lavé par diafiltration avec de l'eau. L'extrait protéinique ainsi purifié est filtré stérilement et éventuellement lyophilisé de manière usuelle connue en soi.

Les analyses biochimiques et les tests immunopharmacologiques ont été effectués sur le lyophilisat. La détermination des acides aminés racémisés lors de l'extraction alcaline a été effectuée selon Nimura N. et Kinoshita T., J. Chromatography, 352, 169-177, 1986. Les différentes racémisations détectées dans les extraits protéiniques (lots I, II et III) sont donnés à titre d'exemple dans le Tableau 2 et exprimés en % de configuration D.

**Tableau 2:**

| Acides aminés racémisés. | | | |
|---|---|---|---|
| Paramètres | % configuration D | | |
| Lots | I | II | III |
| *Acides Aminés racémisés:* | | | |
| Acide Aspartique | 18 | 18 | 20 |
| Sérine | 33 | 35 | 39 |
| Arginine | 8 | 10 | 12 |

Non racé misés: Alanine, Tyrosine, Valine, Phénylalanine et Leucine.

Non détectable: Acide Glutamique, Thréonine, Proline, Méthionine, Isoleucine, Cystéine, Lysine, Histidine et Tryptophane.

Les résultats analytiques figurent dans le Tableau 3.

**Tableau 3:**

| Constituants du lyophilisat. | | | |
|---|---|---|---|
| Paramètres | % Lyophilisat | | |
| Lots | I | II | III |
| *Teneur en protéines:* | | | |
| Protéines (Bradford) | 69,2 | 70,4 | 63,1 |

| *Acides Aminés Protéiniques:* | | | |
|---|---|---|---|
| Asparagine & Acide Aspartique | 7,3 | 7,6 | 7,1 |
| Glutamine & Acide Glutamique | 9,6 | 9,5 | 8,9 |
| Sérine | 2,6 | 2,4 | 2,2 |
| Thréonine | 2,9 | 3,2 | 2,3 |
| Glycine | 3,8 | 3,9 | 3,3 |
| Alanine | 5,9 | 5,6 | 5,6 |
| Arginine | 4,8 | 4,6 | 5,3 |
| Proline | 2,5 | 2,4 | 2,5 |
| Valine | 4,9 | 5,0 | 4,4 |
| Méthionine | 3,1 | 4,0 | 3,4 |
| Isoleucine | 4,1 | 4,0 | 3,5 |
| Leucine | 6,3 | 6,3 | 5,9 |
| Phénylalanine | 3,3 | 3,3 | 3,3 |
| Cystine | ND | ND | ND |
| Lysine | 5,0 | 4,9 | 4,7 |
| Histidine | 1,6 | 1,9 | 1,9 |
| Tyrosine | 3,9 | 4,0 | 3,9 |
| Total | 71,7 | 72,6 | 68,1 |

| *Fractions liées aux protéines:* | | | |
|---|---|---|---|
| Acides gras | 0,9 | 0,9 | 1,0 |
| Glucides | 3,9 | 3,6 | 4,7 |
| Sucres aminés | 1,8 | 1,5 | 2,8 |

| *Autres:* | | | |
|---|---|---|---|
| Lipopolysaccharides (LPS) | 6,1x10⁻⁴ | 8,4x10⁻⁴ | 2,5x10⁻⁴ |
| Acides Déoxyribonucléiques (ADN) | 9,5 | 6,1 | 4,5 |
| Acides aminés libres | ND | ND | ND |
| Eau | 4,8 | 4,9 | 4,8 |

| *Analyses élémentaires:* | | | |
|---|---|---|---|
| Cendres | 6,0 | 7,5 | 7,2 |
| Carbone | 47,1 | 47,0 | 46,3 |
| Hydrogène | 6,7 | 6,7 | 6,6 |
| Azote | 13,6 | 14,0 | 13,6 |
| Soufre | 1,3 | 1,4 | 1,8 |
| ND = Non Détecté | | | |

### Propriétés immunopharmacologiques

Il a été clairement démontré, notamment par des tests in vitro et in vivo sur des modèles animaux, que l'extrait protéinique selon l'invention, possède des propriétés immunomodulatrices et une activité antitumorale.

### 1. Tests immunologiques in vitro.

Les tests immunologiques in vitro ont été réalisés sur des macrophages dérivés de moëlle osseuse et sur des lymphocytes provenant de rate, de plaques de Peyer ou de ganglions mésentériques, prélevés sur des souris C57BL/6.

*Modèles macrophagiques:* L'extrait protéinique stimule les macrophages dans leur capacité d'activer le métabolisme oxydatif du glucose dans la voie des pentoses phosphates et active la production de métabolites de l'azote (test des nitrites). La sécrétion du TNF ainsi que la production des prostaglandines (PGE₂) chez le macrophage sont stimulées par l'extrait protéinique.

Le test des nitrites, présenté à titre d'exemple, a été effectué selon Marletta M. A., Biochemistry, 27, 8706-8711, 1988. Les macrophages dérivés de la moëlle osseuse sont mis en culture en microplaque 170.000 macrophages par puit) en présence de différentes concentrations de l'extrait protéinique (0.1 à 50 µg extrait/mL) ou de lipopolysaccharides de E. coli comme contrôle positif (0.0004 à 0.2 µg LPS/mL). La production de NO₂⁻ dans les surnageants des cultures de macrophages est déterminée avec le réactif de Griess, selon Mauël J. *et al,* Int. J. Immunopharmac., 11,637-645, 1989.

**Tableau 4:**

| Résultats du test des nitrites | | | |
|---|---|---|---|
| µg extrait/mL | nmol NO₂⁻/mL | µg LPS/mL | nmol NO₂⁻/mL |
| 0 | <0,02 | 0 | <0,02 |
| 0,1 | 0,9 ± 0,1 | 0,0004 | 0,1 ± 0,1 |
| 0,39 | 4,1 ± 0,1 | 0,0016 | 0,3 ± 0,1 |
| 1,56 | 7,7 ± 0,2 | 0,0063 | 4,2 ± 0,1 |
| 6,25 | 10,8 ± 0,2 | 0,025 | 6,5 ± 0,4 |
| 25,0 | 13,1 ± 0,8 | 0,1 | 7,1 ± 1,0 |
| 50,0 | 15,1 ± 0,3 | 0,2 | 8,3 ± 0,2 |

Les résultats aux différentes concentrations des produits testés sont présentés dans le Tableau 4 en nmol NO₂-/mL de surnageant cellulaire en fonction de la concentration d'extrait ou de LPS. L'extrait protéinique induit une forte production de NO₂-.

*Modèles lymphocytaires:* Des lymphocytes provenant de rate, de plaques de Peyer ou de ganglions mésentériques sont mis en culture dans des microplaques (5 x 10⁵ cellules par puit) dans des conditions de culture standardisées en présence de différentes concentrations de l'extrait protéinique (1 à 100 µg extrait/mL) ou de lipopolysaccharides de E. coli comme contrôle positif (1 à 100 pg LPS/mL). La prolifération cellulaire induite par les produits est déterminée par le taux d'incorporation de thymidine tritiée dans l'ADN des cellules, selon Louis J. *et al,* Eur. J. Immunol., 9, 841-847, 1979. L'amplitude de la stimulation des lymphocytes par l'extrait protéinique est comparable, à concentration égale, avec celle du contrôle positif pour les 3 sources de lymphocytes. L'extrait protéinique est actif dès 1 µg extrait/mL et montre un maximum d'activité entre 30 et 100 µg extrait/mL.

### 2. Test immunologique in vivo.

L'activité antitumorale de l'extrait protéinique a été mise en évidence in vivo dans un modèle de carcinomatose péritonéale induite chez les rats BD IX.

*Les cellules utilisées:* Les cellules Pro b ont été clonées à partir d'une culture de cellules K12, elles-mêmes isolées d'une tumeur DHD induite par la diméthylhydrazine chez un rat BD IX consanguin, selon: Martin F. *et al,* Int. J. Cancer, 32, 623-627, 1983.

*Induction des carcinomatoses péritonéales:* Les cellules Pro b, injectées par voie intrapéritonéale aux rats syngéniques (10⁶ cellules/rat), donnent naissance en une dizaine de jours à de nombreux nodules solides qui apparaissent dans l'épiploon ou le mésentère au niveau des taches laiteuses (milky spots), puis envahissent progressivement la cavité péritonéale, selon Lagadec P. *et al,* Invasion and Metastasis, 7, 83-95, 1987. Une ascite hémorragique apparaît 4 à 5 semaines plus tard et la totalité des rats meurt en 8 à 12 semaines.

*Induction des métastases pulmonaires:* L'injection de 7 x 10⁶ cellules Pro b dans la veine fémorale provoque des métastases pulmonaires qui envahissent les lobes et tous les rats meurent en 6 à 10 semaines.

*Traitement des carcinomatoses péritonéales et pulmonaires:* L'immunothérapie commence 14 jours après l'injection des cellules tumorales lorsque les carcinomatoses sont macroscopiques. Le traitement consiste en injections intrapéritonéales de l'extrait protéinique à 10 mg par kg de poids corporel. Les rats reçoivent au total 5 injections, espacées de 3 à 5 jours. Chaque expérience comporte un groupe témoin et un groupe traité de 10 (voire 12) rats, tous numérotés.

*Résultats:* Les rats sont sacrifiés et autopsiés 6 semaines après l'injection des cellules. Le volume des carcinomatoses péritonéales est évalué en aveugle et les rats sont classés par ordre de carcinomatose croissante. Une échelle des carcinomatoses est établie selon le nombre et la taille des nodules observés. Le volume des ascites hémorragiques est mesuré par double pesée. La classification des métastases pulmonaires a été effectuée après observation microscopique des poumons et les rats ont été placés dans deux classes: avec ou sans métastases.

Les résultats observés montrent que dans huit expériences portant sur 82 rats traités, 41 rats ne présentaient aucun nodule à l'autopsie (40 à 60 % des rats par expérience); chez les autres rats la croissance des nodules était significativement inhibée. De plus, 78 des 82 rats traités ne présentaient pas d'ascites. Tous les rats non traités avaient des tumeurs et des ascites hémorragiques. Ces résultats sont confirmés par ceux obtenus dans une expérience de survie: sur 10 rats traités avec l'extrait protéinique, 3 rats ont survécu jusqu'à 10, 18 et 27 mois après l'injection des cellules cancéreuses et n'ont pas présenté de tumeur à l'autopsie. Tous les rats du groupe témoin sont morts de leurs tumeurs 3 mois après l'injection des cellules.

De plus, les résultats obtenus lors de 2 expériences sur la croissance des métastases pulmonaires ont permis de démontrer que l'extrait protéinique a un effet systémique. En effet, sur 20 rats traités par injection intrapéritonéale, 13 rats présentent une complète inhibition de la croissance des métastases pulmonaires. L'effet antitumoral de l'extrait protéinique est obtenu sur des métastases induites par des cellules cancéreuses du colon et également par les tumeurs dissociées. Aucun signe de toxicité n'a été observé durant le traitement.

### 3. Toxicité aigüe

L'extrait protéinique a une faible toxicité. Des doses uniques allant jusqu'à 300 mg par kg, injectées intrapéritonéalement, sont bien tolérées par la souris.

### 4. Administration

L'administration de l'extrait protéinique est effectuée par injection de préférence intrapéritonéale du lyophilisat dissous dans du soluté physiologique, par exemple. D'autres formes galéniques peuvent également être envisagées, par exemple, des formes administrables par voie orale, rectale ou topique.

## Revendications

1. Extrait à base de protéines bactériennes, dont certains acides aminés sont racémisés et les résidus asparagine et glutamine sont partiellement désaminés, comportant un mélange de polyanions bactériens acides ayant un poids moléculaire de 10'000 à 1'000'000 et un point isolélectrique compris entre 2,5 et 5,5, dans lequel la somme pondérale des acides aminés constitutifs représente au moins 50% dudit extrait, et susceptible d'être obtenu par le procédé qui comporte la culture de bactéries en milieu liquide, l'élimination du milieu de culture et la mise en suspension de ces bactéries dans un milieu aqueux, puis l'extraction alcaline de cette suspension bactérienne et la purification de l'extrait protéinique, l'extraction alcaline étant effectuée en présence d'une source aqueuse diluée de ions OH⁻ et à un pH stable compris entre 11 et 13, la baisse de ce pH au cours de ladite extraction n'étant pas supérieure à 0,4.

2. Extrait à base de protéines bactériennes selon la revendication 1, caractérisé par le fait que ladite somme pondérale des acides aminés est comprise entre 55 et 85%, que la teneur en lipopolysaccharides est inférieure à environ 2 x 10⁻³ %, par le fait qu'il comporte au plus environ 2% d'acides aminés libres, au plus environ 8% de glucides, au plus environ 4% de sucres aminés et au plus environ 15% d'acide désoxyribonucléique.

3. Extrait à base de protéines bactériennes selon la revendication 1, caractérisé par le fait que les acides aminés racémisés sont au moins la sérine ayant de 25 à 45% de configuration D, l'acide aspartique de 10 à 30% et l'arginine de 3 à 20 %.

4. Procédé pour la préparation d'un extrait à base de protéines bactériennes selon la revendication 1, qui comporte la culture de bactéries en milieu liquide, l'élimination du milieu de culture et la mise en suspension de ces bactéries dans un milieu aqueux, puis l'extraction alcaline de cette suspension bactérienne et la purification de l'extrait protéinique, l'extraction alcaline étant effectuée en présence d'une source aqueuse diluée de ions OH⁻ et à un pH stable compris entre 11 et 13, la baisse de ce pH au cours de ladite extraction n'étant pas supérieure à 0,4.

5. Procédé selon la revendication 4, caractérisé par le fait que les bactéries cultivées sont des Escherichia coli, par exemple la souche I-1147 déposée à la Collection Nationale de Cultures de Microorganismes, que la source de ions OH⁻ est du NaOH 0,01 à 1%, que le pH est compris entre 12,0 et 12,5 et que la température est maintenue entre 30 et 45°C pendant l'extraction, la durée de celle-ci étant de quelques heures à une semaine.

6. Procédé selon la revendication 4 ou la revendication 5, caractérisé par le fait que l'extrait protéinique obtenu par extraction alcaline est soumis à une purification, de telle sorte que la teneur en LPS de l'extrait protéinique soit inférieure à 2 x 10⁻³ %, cette purification comportant une ou plusieurs étapes d'ultrafiltration, de traitement avec un détergent non-ionique, de chromatographie et/ou de filtration stérile, ainsi qu'une lyophilisation.

7. Composition pharmaceutique contenant à titre d'ingrédient actif une quantité thérapeutiquement acceptable de l'extrait à base de protéines bactériennes modifiées selon l'une des revendications 1 à 3.

8. Composition pharmaceutique selon la revendication 7, ayant des propriétés immunomodulatrices.

9. Composition pharmaceutique selon la revendication 7 ayant un effet thérapeutique anti-cancéreux.

10. Composition thérapeutique selon l'une des revendications 7 à 9, caractérisée par le fait qu'elle est préparée sous une forme injectable ou sous une forme administrable par voie orale, rectale ou topique.

## Patentansprüche

1. Extrakt aus bakteriellen Proteinen, von denen bestimmte Aminosäuren racemisiert und die Asparagin- und Glutaminreste teilweise desaminiert sind, bestehend aus einer Mischung von sauren bakteriellen Polyanionen mit einem Molekulargewicht von 10 000 bis 1 000 000 und einem isoelektrischen Punkt zwischen 2,5 und 5,5, worin die Gesamtmenge der konstitutionellen Aminosäuren zumindest 50 % des benannten Extrakts darstellt, und erhältlich durch das Verfahren, das aus der Bakterienkultur in flüssigem Medium, der Entfernung des Kulturmediums und der Aufschlämmung dieser Bakterien in einem wässrigen Medium, sodann der alkalischen Extraktion dieser Bakterienaufschlämmung und der Reinigung des Proteinextrakts besteht, wobei die alkalische Extraktion in Gegenwart einer verdünnten wässrigen Quelle von OH⁻-Ionen und bei einem stabilen pH-Wert zwischen 11 und 13 erfolgt und die pH-Abnahme im Verlaufe der benannten Extraktion nicht mehr als 0,4 beträgt.

2. Extrakt aus bakteriellen Proteinen gemäss Anspruch 1, dadurch gekennzeichnet, dass die benannte Gesamtmenge der Aminosäuren zwischen 55 und 85 % liegt, dass der Gehalt an Lipopolysacchariden unterhalb von etwa 2 - 10⁻³ % liegt, dass der Extrakt höchstens etwa 2 % freie Aminosäuren, höchstens etwa 8 % Kohlenhydrate, höchstens etwa 4 % Aminozucker und höchstens etwa 15 % Desoxyribonucleinsäure enthält.

3. Extrakt aus bakteriellen Proteinen gemäss Anspruch 1, dadurch gekennzeichnet, dass die racemisierten Aminosäuren zumindest Serin mit 25 bis 45 %, Asparaginsäure mit 10 bis 30 % und Arginin mit 3 bis 20 % der D-Konfiguration sind.

4. Verfahren zur Herstellung eines Extrakts aus bakteriellen Proteinen gemäss Anspruch 1, das aus der Bakterienkultur in flüssigem Medium, der Entfernung des Kulturmediums und der Aufschlämmung dieser Bakterien in einem wässrigen Medium, sodann der alkalischen Extraktion dieser Bakterienaufschlämmung und der Reinigung des Proteinextrakts besteht, wobei die alkalische Extraktion in Gegenwart einer verdünnten wässrigen Quelle von OH⁻-Ionen und bei einem stabilen pH-Wert zwischen 11 und 13 erfolgt und die pH-Abnahme im Verlaufe der benannten Extraktion nicht mehr als 0,4 beträgt.

5. Verfahren gemäss Anspruch 4, dadurch gekennzeichnet, dass die kultivierten Bakterien *Escherichis coli* sind, zum Beispiel der in der Nationalen Kulturensammlung von Mikroorganismen deponierte Stamm I-1147, dass die Quelle der OH⁻-Ionen 0,01- bis 1%ige NaOH ist, dass der pH-Wert zwischen 12,0 und 12,5 liegt und dass die Temperatur während der Extraktion zwischen 30 und 45 °C gehalten wird, wobei die Dauer dieser Extraktion zwischen einigen Stunden und einer Woche beträgt.

6. Verfahren gemäss Anspruch 4 oder Anspruch 5, dadurch gekennzeichnet, dass der durch alkalische Extraktion erhaltene Proteinextrakt einer Reinigung unterworfen wird, so dass der LPS-Gehalt des Proteinextrakts weniger als 2 · 10⁻³ % beträgt, wobei diese Reinigung einen oder mehrere Schritte der Ultrafiltration, der Behandlung mit einem nichtionischen Detergens, der Chromatographie und/oder der Sterilfiltration sowie eine Gefriertrocknung umfasst.

7. Pharmazeutische Zusammensetzung, die als aktiven Bestandteil eine therapeutisch annehmbare Menge des Extrackts aus gemäss einem der Ansprüche 1 bis 3 abgewandelten bakteriellen Proteinen enthält.

8. Pharmazeutische Zusammensetzung gemäss Anspruch 7 mit immunitätsverändernden Eigenschaften.

9. Pharmazeutische Zusammensetzung gemäss Anspruch 7 mit krebstherapeutischer Wirkung.

10. Therapeutische Zusammensetzung gemäss einem der Ansprüche 7 bis 9, dadurch gekennzeichnet, dass sie in einer einspritzbaren oder oral, rektal oder lokal verabreichbaren Form hergestellt wird.

## Claims

1. An extract based on modified bacterial proteins, whose certain amino acids are racemized and the asparagine and glutamine residues are partly desaminated, including a mixture of bacterial acid polyanions having a molecular weight in the range from 10'000 to 1'000'000 and an isoelectric point in the range from 2.5 to 5.5, and in which the added weights of the constituent amino acids amount to at least 50% of said extract, and obtainable by the process with includes cultivating bacteria in a liquid medium, eliminating the cultivation medium and suspending these bacteria in an aqueous medium, then carrying out the alkaline extraction of this bacterial suspension and a purification of the bacterial extract, the alkaline extraction being carried out in the presence of a diluted aqueous source of OH⁻ ions at a stable pH comprised between 11 and 13, the decrease in pH during said extraction not exceeding 0.4.

2. An extract based on bacterial proteins according to claim 1, characterized in that said added weights of the amino acids is in the range from 55 to 85%, in that the lipopolysaccharides content is lesser than about 2x10%, in that it includes at the most about 2% of free amino acids, at the most about 8% of glucides, at the most about 4% of amino sugars and at the most about 15% of desoxyribonucleic acid.

3. An extract based on bacterial proteins according to claim 1, characterized in that: the racemized amino acids are at least serine having 25 to 45% D configuration, aspartic acid 10 to 30%, and arginine 3 to 20%.

4. A process for the preparation of an extract based on bacterial proteins according to claim 1, which includes cultivating bacteria in a liquid medium eliminating the cultivation medium and suspending these bacteria in an aqueous medium, then carrying out the alkaline extraction of this bacterial suspension and a purification of the bacterial extract, the alkaline extraction being carried out in the presence of a diluted aqueous source of OH ions at a stable pH comprised between 11 and 13, the decrease in pH during said extraction not exceeding 0.4.

5. A process according to claim 4, characterized in that the cultivated bacteria are Escherichia coli, for example the strain I-1147 deposited at the Collection Nationale de Cultures de Microorganismes, in that the source of OH ions is 0.01 to 1% NaOH, in that the pH is within the range from 12.0 to 12.5 and in that the temperature is maintained between 30 and 45°C during the extraction, the duration of the latter ranging from several hours to one week.

6. A prcess according to claim 4 or claim 5, characterized in that the protein extract obtained by alkaline extraction is subjected to a purification, so that the LPS content of the protein extract be lesser than 2x10%, this purification including one or several steps of ultrafiltration, treatment with a non ionic surfactant, chromatography and/or sterile filtration, as well as lyophilization.

7. A pharmaceutical composition containing as the active ingredient a therapeutically acceptable amount of the extract based on bacterial proteins modified according to claims 1 to 3.

8. A pharmaceutical composition according to claim 7, having immunomodulator properties.

9. A pharmaceutical composition according to claim 7, having an anticancer therapeutic effect.

10. A pharmaceutical composition according to one of claims 7 to 9, characterized in that it is prepared in an injectable form or in a dosage form for oral, rectal or topical administration.
